# EUROPEAN PATENT SPECIFICATION

(11) **EP 2 604 169 B1**
(45) Date of publication and mention of the grant of the patent: **14.10.2015**
(21) Application number: 11826665.9
(22) Date of filing: 23.08.2011
(51) Int. Cl.: A61B 1/00, A61B 1/04

(54) **FLUORESCENCE OBSERVATION APPARATUS**
FLUORESZENZBEOBACHTUNGSGERÄT
DISPOSITIF D'OBSERVATION DE FLUORESCENCE

(30) Priority: 22.09.2010 JP 2010212486
(43) Date of publication of application: 19.06.2013
(73) Proprietor: Olympus Corporation, Shibuya-ku Tokyo 151-0072 (JP)
(72) Inventor: MORISHITA, Koki, Tokyo 151-0072 (JP); HASEGAWA, Akira, Tokyo 151-0072 (JP)
(74) Representative: Schicker, Silvia
(86) International application number: PCT/JP2011/068905
(87) International publication number: WO 2012/039219

(56) References cited:
- WO-A1-99/53832
- WO-A1-2009/057774
- JP-A- 2006 141 734
- JP-A- 2007 307 278
- JP-A- 2008 167 922
- JP-A- 2010 220 895
- US-A1- 2004 064 016
- US-A1- 2009 093 728

## Description

### {Technical Field}

The present invention relates to fluorescence observation apparatus.

### {Background Art}

In the related art, research results that have been obtained show that the number and the size of ACF (aberrant crypt foci) formed in recta have a strong correlation with a future incidence of cancer. In such research, a method is employed, in which, for example, the ductal structure is made visible using methylene blue, and the ACF are checked one-by-one while counting the number thereof by using a magnifying endoscope or the like (for example, see Non Patent Literature 1).

### {Citation List}

### {Non Patent Literature}

{NPL 1} Cancer Epidemiol Biomarkers Prev., May 2008; 17(5): 1155-62

WO 99/53832 A1 discloses an imaging system for fluorescence endoscopy comprising a light source that produces fluorescence excitation light, a dual channel fluorescence camera and a counter circuit for determining the number of pixels in the image signals having magnitudes greater than or less than reference thresholds.

US 2009/093728 A1 discloses a device for imaging and ablating medical target cells within vascular or lymphatic systems. The device comprises a laser able to provide both excitation and ablation energy, electromagnetic energy sensors for measuring fluorescence of one or more targets and one or more vessel insertion units for counting numbers of one or more targets.

US 2007/270652 A2 discloses an endoscope system comprising an excitation light source that emits excitation light, a variable spectroscopic element and an image capture portion for picking up fluorescence.

### {Summary of Invention}

### {Technical Problem}

However, observing the entire rectum and checking the ACF one-by-one by using a magnifying endoscope or the like as in the related art can be a problem in that the ACF can possibly get overlooked and that the observation time is too long since the observation must be meticulously performed in every corner to prevent the ACF from getting overlooked. Moreover, the definition for determining whether an observed object is ACF or not depends on the observer's subjective assessment, which is also a problem in that it lacks accuracy.

The present invention has been made in view of these circumstances, and an object thereof is to provide a fluorescence observation apparatus that can readily count Lhe number of ACF without overlooking the existence thereof and that can achieve reduced observation time.

### {Solution to Problem}

In order to solve the aforementioned problems, the present invention provides a fluorescence observation apparatus according to claim 1.

According to this aspect, when the illuminating section inserted into the body cavity, to which fluorescent probes (referred to as "iNOS-sensitive fluorescent probes" hereinafter) or fluorescent probes (referred to as "NO-sensitive fluorescent probes" hereinafter) whose fluorescence characteristics change by reacting with NO produced by activation of iNOS are applied in advance, emits the excitation light onto the inner wall of the body cavity, the image acquisition section acquires the image of the fluorescence generated by the excitation of the fluorescent probes sprayed into the inner wall of the body cavity, thereby acquiring the image information. Then, the counting section counts the number of fluorescence generation sites included in the image information and generated from accumulation sites of the fluorescent probes.

In this case, according to "World Journal of Gastroenterology", 2003, 9(6), p.1246-1250, iNOS (nitric oxide synthase) is known to occur abundantly in ACF (aberrant crypt foci).

Therefore, by exiting the iNOS-sensitive fluorescent probes or NO-sensitive fluorescent probes in the inner wall of the body cavity with excitation light, strong fluorescence can be obtained from areas with an abundance of iNOS, that is, areas with ACF. As a result, the number of ACF can be readily counted by the counting section without overlooking the existence of ACF, thereby achieving reduced observation time.

In the above aspect, the fluorescence observation apparatus may further include moving means configured to move the illuminating section and the image acquisition section relative to the inner wall of the body cavity.

With this configuration, the image information can be acquired over a wide area along the inner wall of the body cavity while performing observation.

In the above aspect, the fluorescence observation apparatus further includes a fluorescent-probe discharging section that sprays the fluorescent probe to the inner wall of the body cavity.

This configuration eliminates the need for an additional member for supplying the fluorescent probes to the biological organism.

In the above aspect, the counting section may count the number of fluorescence generation sites existing in an area between 0.4 and 0.8 from the center of the image based on the image information acquired by the image acquisition section when the distance from the center to an edge of the image is defined as 1.

With this configuration, the effect of parallax and noise can be reduced, thereby preventing an ACF counting error.

In the above aspect, the fluorescence observation apparatus may further include a fluorescence-intensity measuring section that measures the fluorescence intensity at each fluorescence generation site, and a fluorescence-intensity classifying section that classifies the number of fluorescence generation sites counted by the counting section in accordance with the fluorescence intensity at each fluorescence generation site measured by the fluorescence-intensity measuring section.

With this configuration, the degree of malignancy of each ACF can be determined on the basis of the magnitude of the fluorescence intensity at the corresponding fluorescence generation site measured by the fluorescence-intensity measuring section. For example, if the fluorescence intensity is high, the degree of malignancy of the ACF is high. Therefore, by using the fluorescence-intensity classifying section to classify the number of fluorescence generation sites in accordance with the fluorescence intensity, the future risk for cancer development or the like can be evaluated.

In the above aspect, the fluorescence observation apparatus may further include a fluorescence-size measuring section that measures the size of each fluorescence generation site, and a fluorescence-size classifying section that classifies the number of fluorescence generation sites counted by the counting section in accordance with the size of each fluorescence generation site measured by the fluorescence-size measuring section.

With this configuration, the degree of malignancy of each ACF can be determined on the basis of the size of the corresponding fluorescence generation site measured by the fluorescence-size measuring section. Therefore, by using the fluorescence-size classifying section to classify the number of fluorescence generation sites in accordance with the size thereof, the future risk for cancer development or the like can be evaluated.

According to the invention, the fluorescence observation apparatus further includes a fluorescence-pattern determining section that detects a pattern of each fluorescence generation site and determines whether or not the pattern matches a pattern of ACF, and a correcting section that subtracts the number of fluorescence generation sites determined as not satisfying a pattern condition of the ACF by the fluorescence-pattern determining section from the number of fluorescence generation sites counted by the counting section.

With this configuration, bright spots, other than ACF, occurring due to noise or the like are removed in accordance with the pattern of each fluorescence generation site detected by the fluorescence-pattern determining section, thereby allowing for more accurate detection of ACF. Therefore, by using the correcting section to correct the counted number of fluorescence generation sites so as to accurately ascertain the number of ACF, the future risk for cancer development or the like can be finely evaluated. Since ACF have, for example, a pattern formed of a group of hollow annular sections with high brightness, the ACF pattern condition may be set, for example, on the basis of a ratio between bright areas and dark areas within the pattern of each fluorescence generation site.

In the above aspect, the fluorescence observation apparatus may further include an image generating section that generates a two-dimensional image of the fluorescence generation sites counted by the counting section, and a pattern classifying section that classifies each fluorescence generation site in accordance with a pattern thereof on the basis of the two-dimensional image generated by the image generating section.

With this configuration, the degree of malignancy of each ACF can be determined on the basis of the pattern of the corresponding fluorescence generation site in the two-dimensional image generated by the image generating section, and the pattern classifying section can be used for the classification, thereby effectively using it for a diagnostic examination.

In the above aspect, the illuminating section may emit reference excitation light having a wavelength characteristic different from that of the excitation light, and the fluorescence observation apparatus may further include an image generating section that generates a two-dimensional fluorescence image and a two-dimensional reference fluorescence image on the basis of the image information corresponding to the excitation light and image information corresponding to the reference excitation light that are acquired by the image acquisition section, and that corrects the fluorescence image acquired using the excitation light by using the reference fluorescence image acquired using the reference excitation light.

With this configuration, since the fluorescence image acquired using the excitation light has fluorescence generation sites, generated by the excitation of the fluorescent probes, and noise, and the reference fluorescence image acquired using the reference excitation light only has noise, the image generating section can remove the noise from the fluorescence image so that the counting section can accurately count the number of ACF. The correction performed by the image generating section may be subtraction processing or division processing.

In the above aspect, the fluorescence observation apparatus may further include an image combining section that obtains a still composite image by combining multiple sets of image information acquired by the image acquisition section while moving the image acquisition section using the moving means, and the counting section may count the number of fluorescence generation sites with predetermined brightness or higher within the still composite image obtained by the image combining section.

With this configuration, based on the still composite image obtained by the image combining section, the number of ACF with a particularly high degree of malignancy can be ascertained in correspondence with the positions and the sizes thereof.

In the above aspect, the fluorescence observation apparatus may further include a reference line that is provided within the image information acquired by the image acquisition section and that moves together with the image acquisition section, and when the image acquisition section is moved by the moving means, the counting section may count the number of fluorescence generation sites that cross over the reference line within the image information acquired by the image acquisition section.

By using the counting section to count the number of fluorescence generation sites every time a fluorescence generation site crosses over the reference line, the number and the positions of the ACF can be ascertained while the image acquisition section acquires the image of the inner wall of the body cavity, thereby achieving reduced observation time.

### {Advantageous Effects of Invention}

The present invention advantageously readily counts the number of ACF without overlooking the existence thereof and achieves reduced observation time.

### {Brief Description of Drawings}

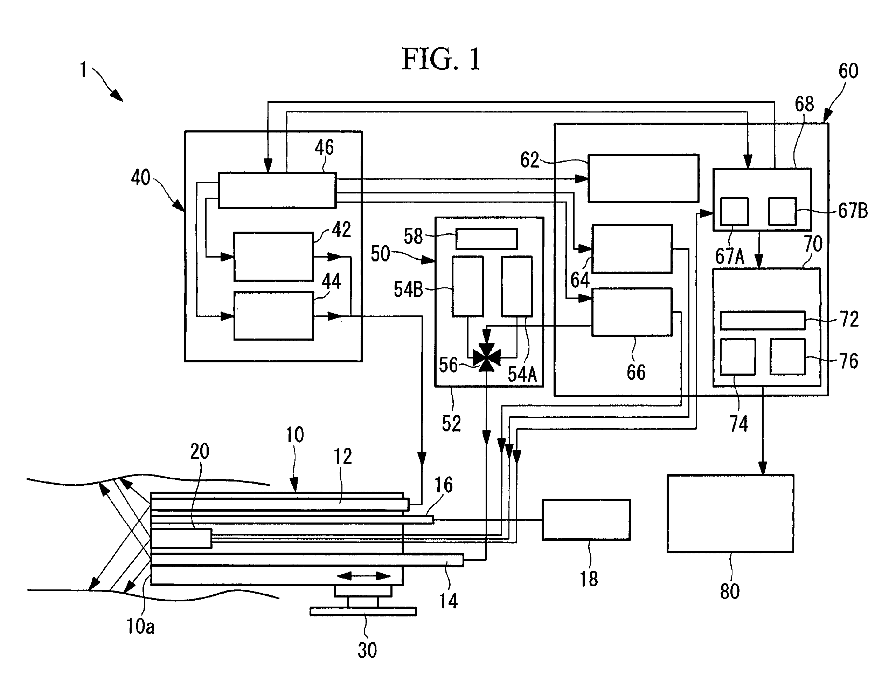

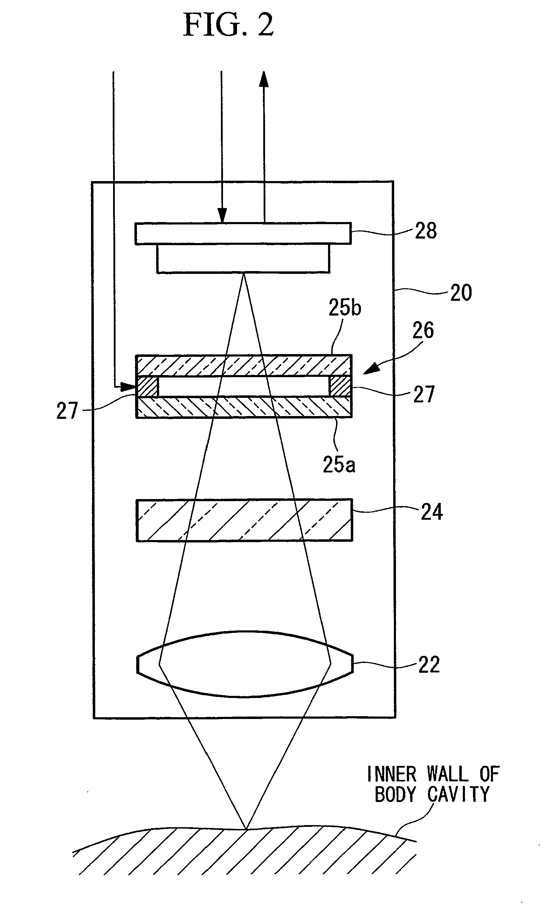

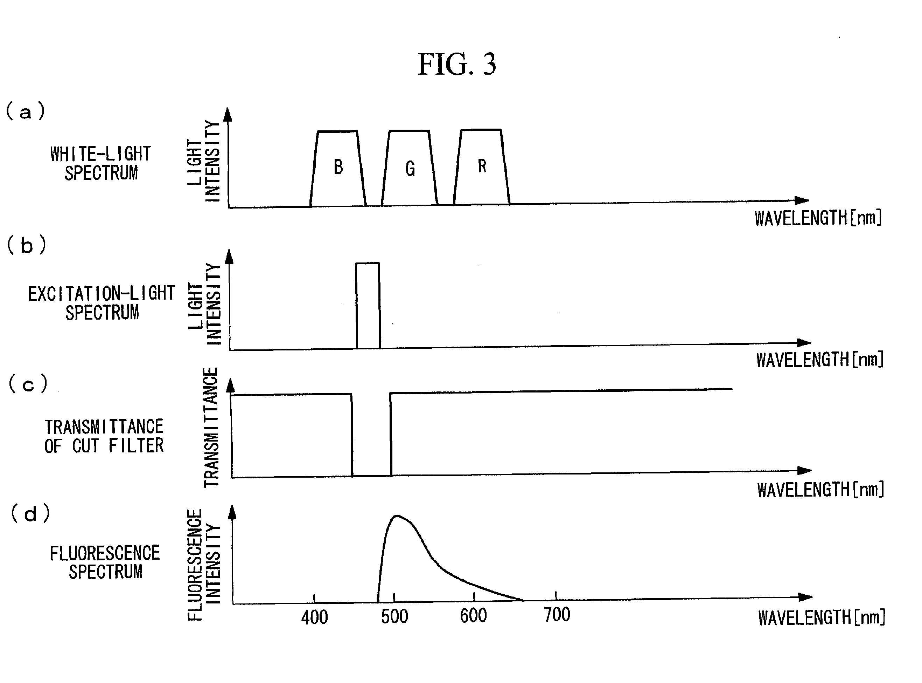

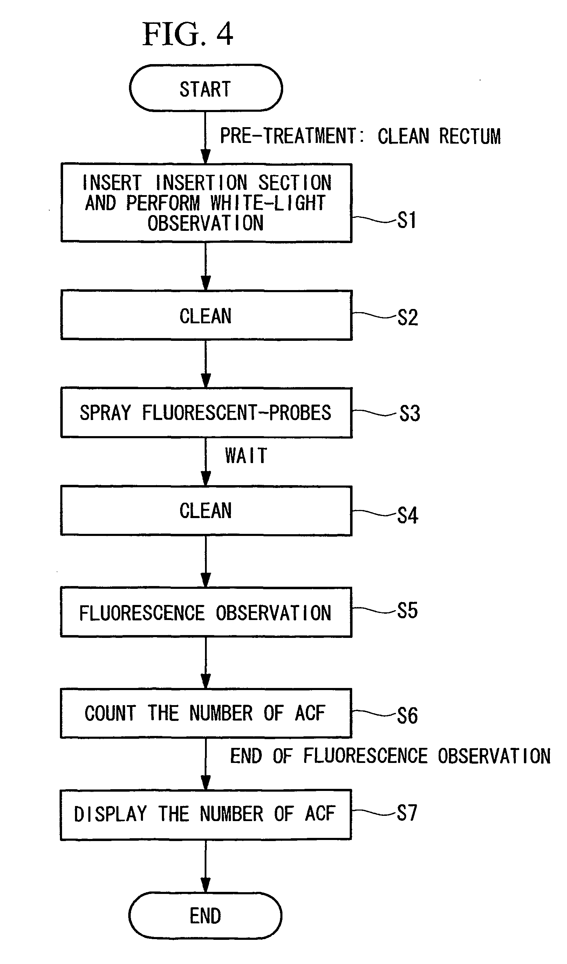

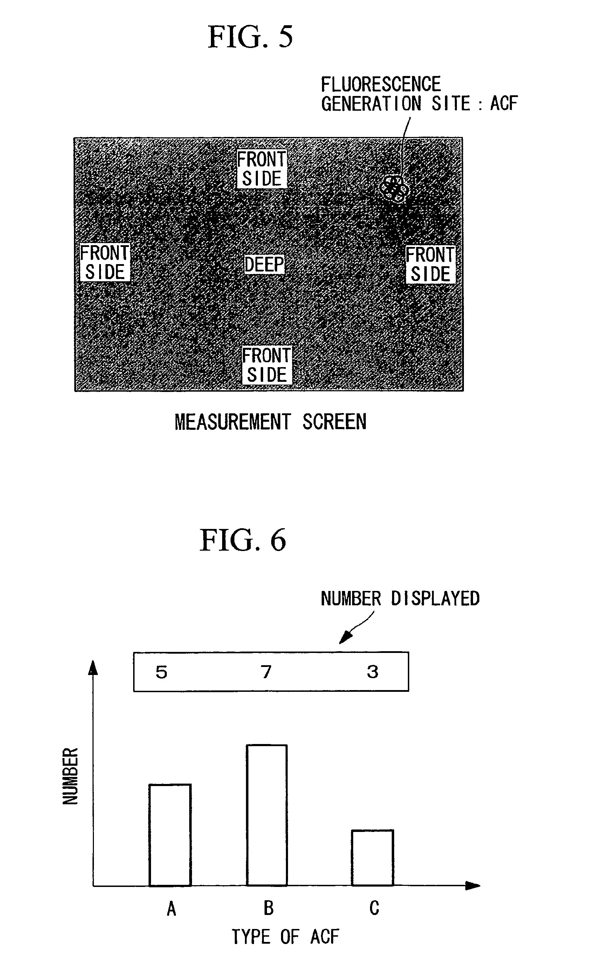

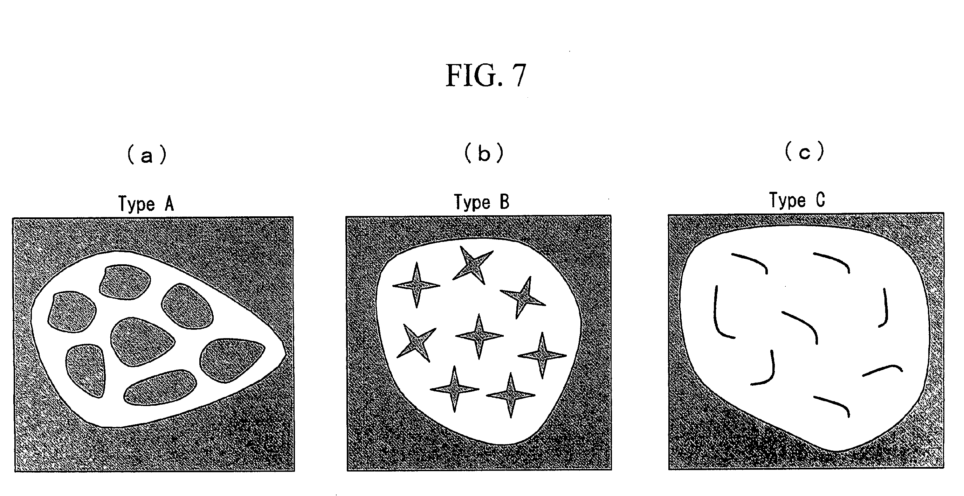

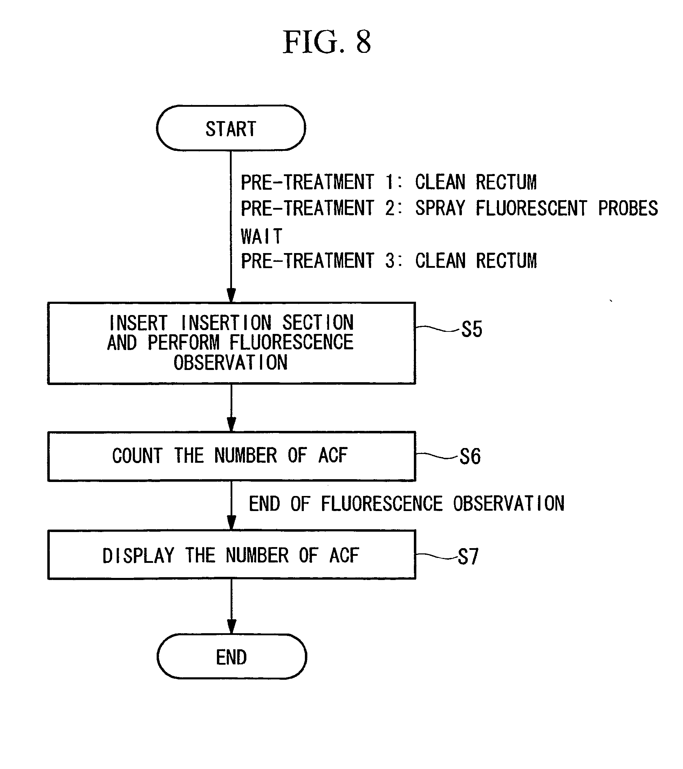

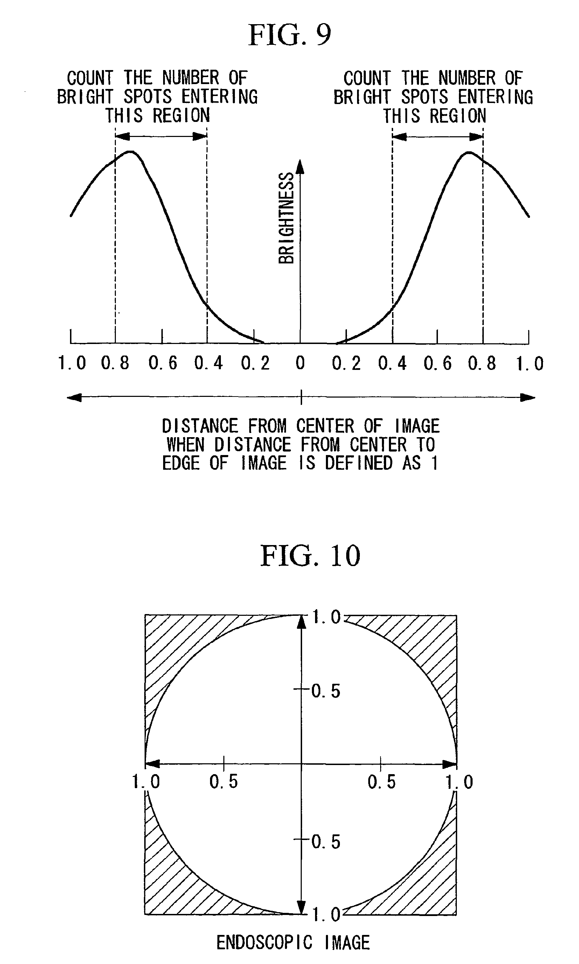

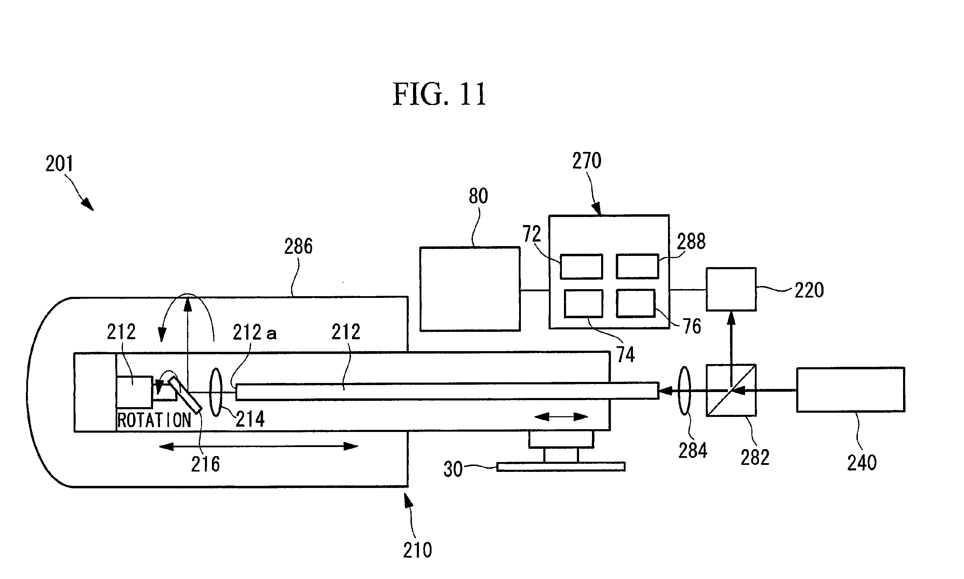

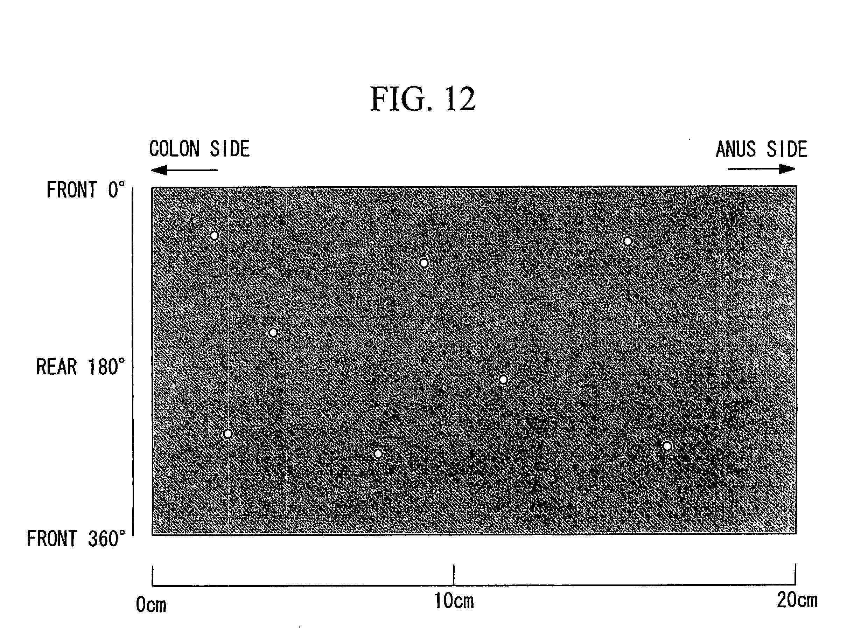

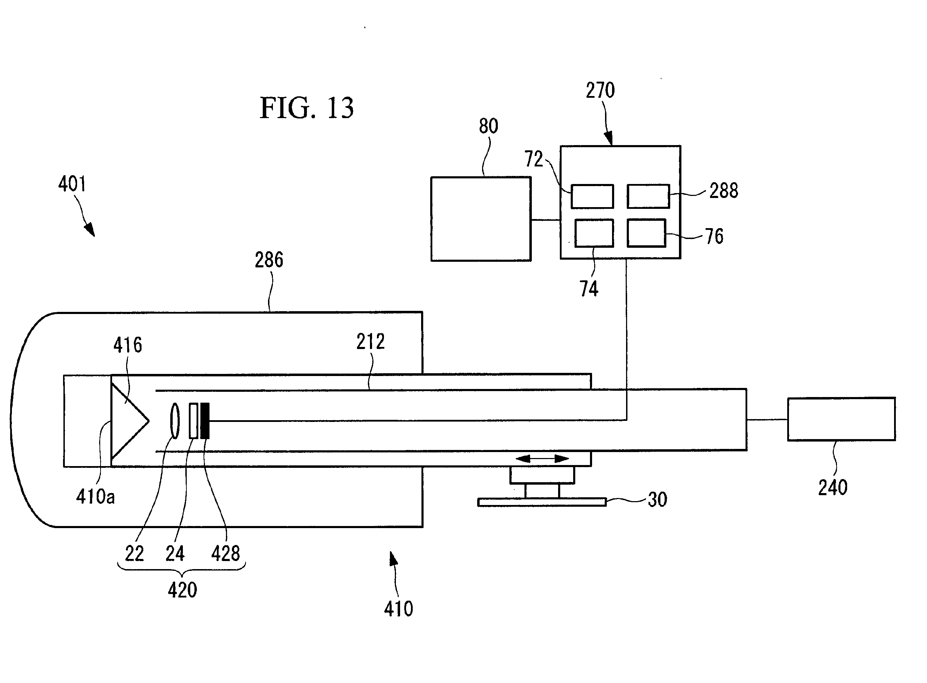

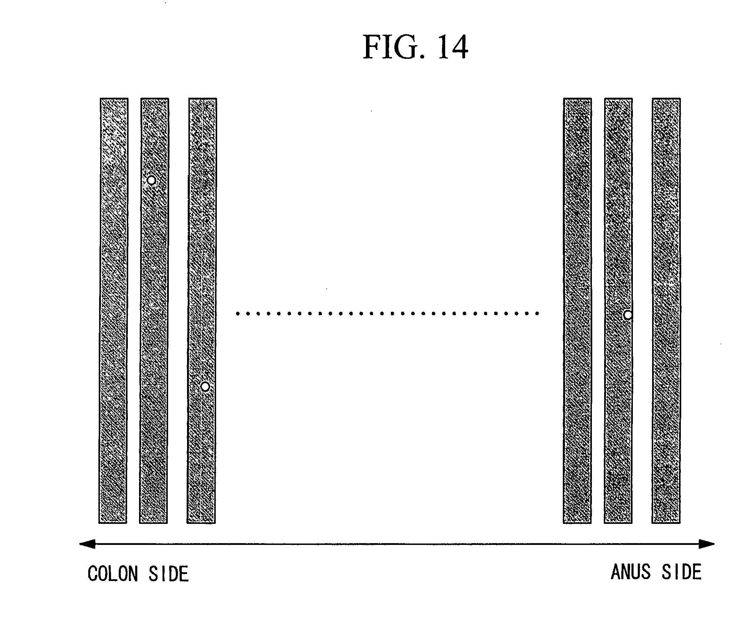

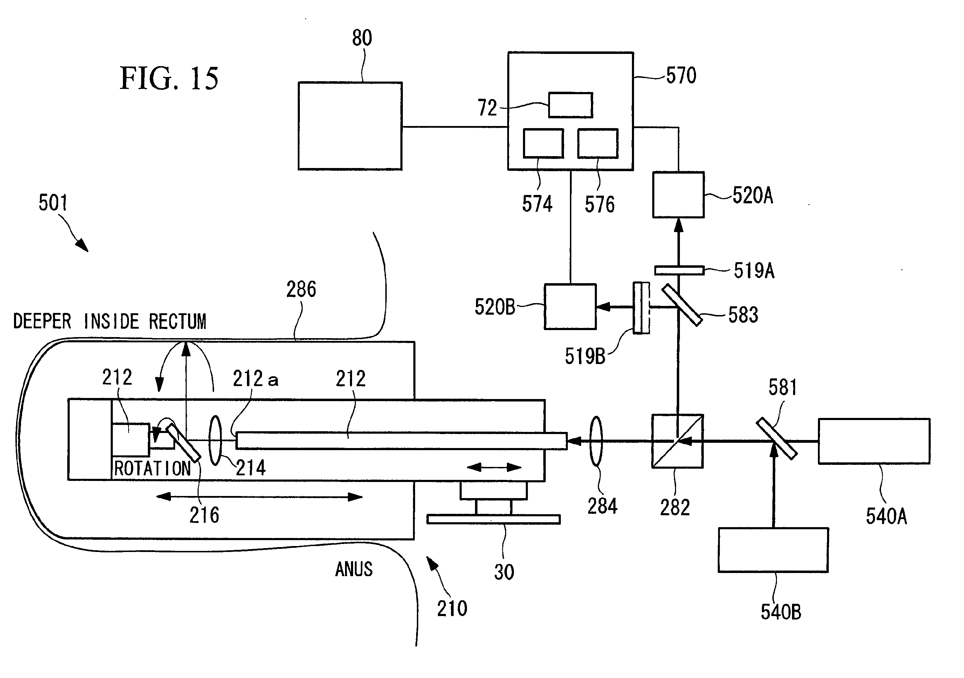

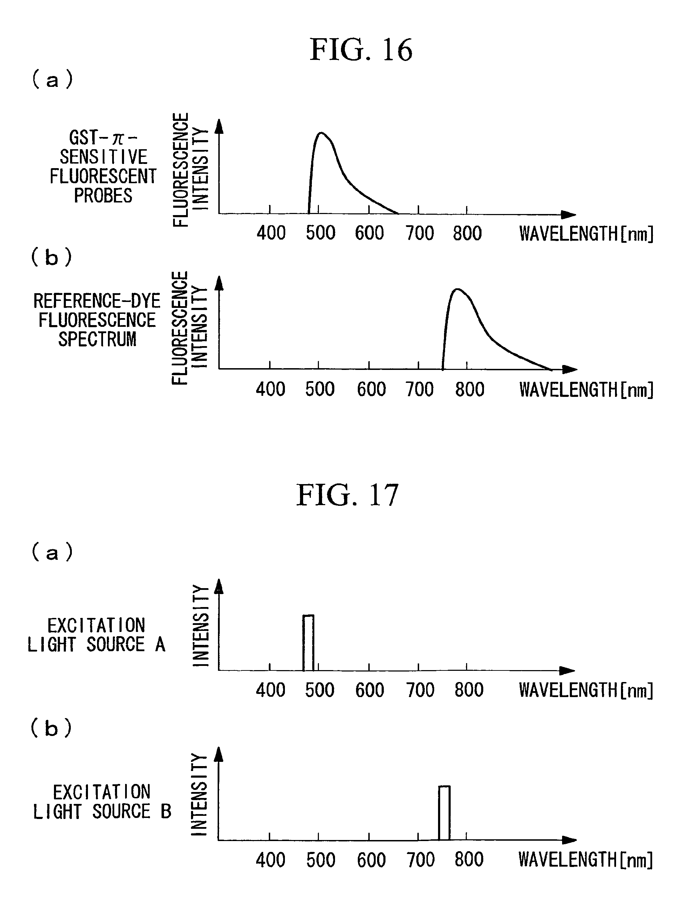

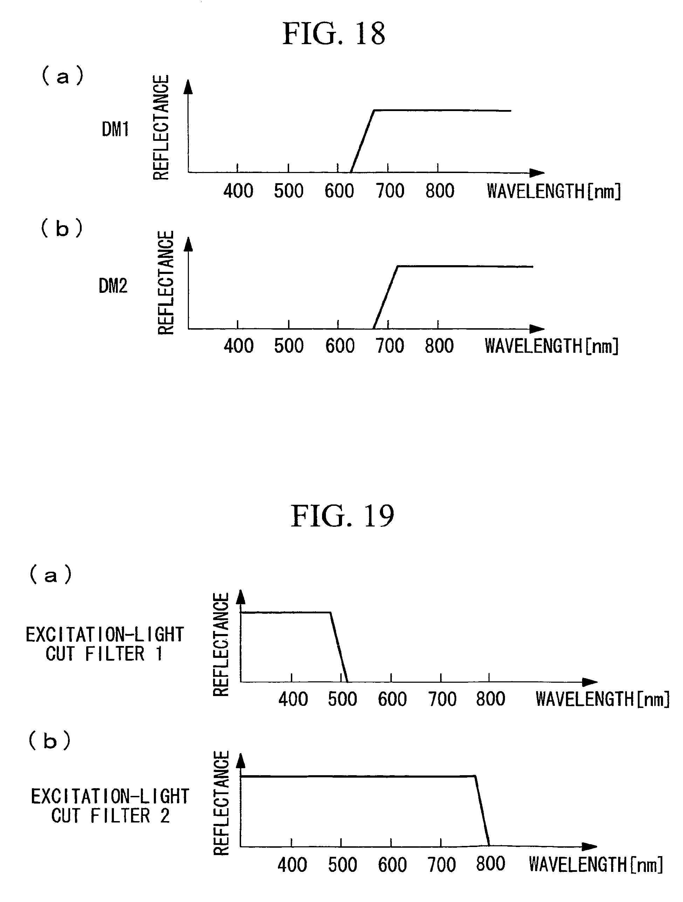

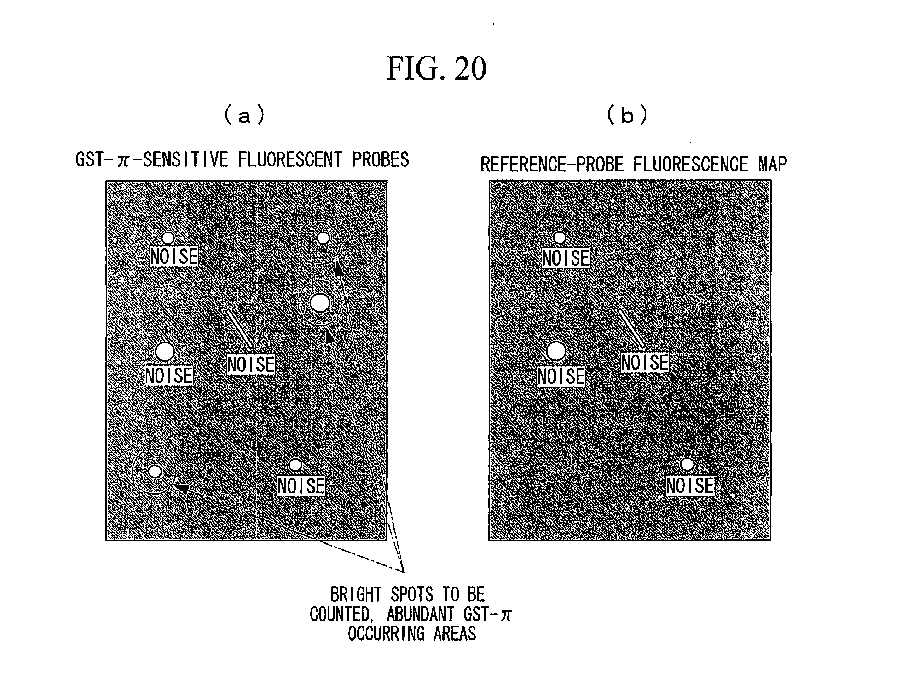

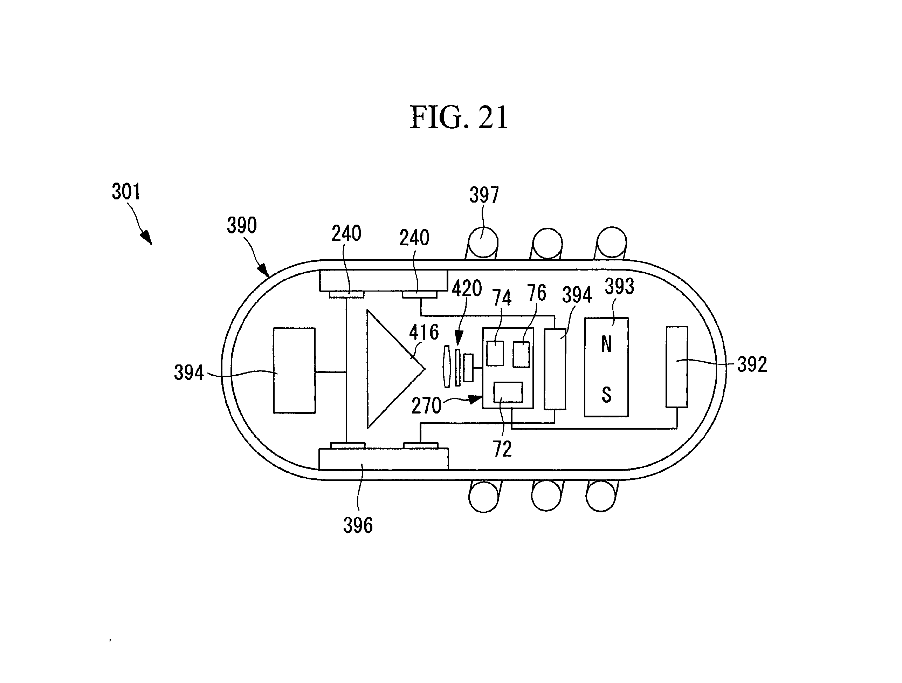

{Fig. 1} Fig. 1 is a schematic configuration diagram of a fluorescence observation apparatus according to a first embodiment of the present invention.
{Fig. 2} Fig. 2 is an enlarged schematic configuration diagram of an image acquisition unit in Fig. 1.
{Fig. 3} Fig. 3 includes part (a) illustrating a wavelength characteristic of illumination light, part (b) illustrating a wavelength characteristic of excitation light, part (c) illustrating a transmittance characteristic of an excitation-light cut filter, and part (d) illustrating a wavelength characteristic of fluorescence from GST-n-sensitive fluorescent probes.
{Fig. 4} Fig. 4 is a flow chart illustrating the operation of the fluorescence observation apparatus in Fig. 1.
{Fig. 5} Fig. 5 is a diagram illustrating an image displayed on a display unit in Fig. 1.
{Fig. 6} Fig. 6 is a diagram illustrating a graph in which the number of ACF is classified in accordance with the fluorescence intensity by a fluorescence-intensity classifying section in Fig. 1.
{Fig. 7} Fig. 7 includes part (a) illustrating the structure of ACF with a circular pit pattern, part (b) illustrating the structure of ACF with a starfish-like pit pattern, and part (c) illustrating the structure of ACF with a collapsed pit pattern.
{Fig. 8} Fig. 8 is a flow chart illustrating the operation of a fluorescence observation apparatus with an alternative configuration according to the first embodiment of the present invention.
{Fig. 9} Fig. 9 is a diagram illustrating the relationship between the distance from the center of an image and the brightness thereof when the distance from the center to an edge of the image is defined as 1.
{Fig. 10} Fig. 10 is a diagram illustrating an image acquired by the fluorescence observation apparatus in Fig. 1.
{Fig. 11} Fig. 11 is a schematic configuration diagram of a fluorescence observation apparatus according to a second embodiment of the present invention.
{Fig. 12} Fig. 12 is a diagram illustrating a still composite image obtained by an image combining section in Fig. 11.
{Fig. 13} Fig. 13 is a schematic configuration diagram of a fluorescence observation apparatus according to a modification of the second embodiment of the present invention.
{Fig. 14} Fig. 14 is a diagram illustrating a still composite image obtained by an image combining section in Fig. 13.
{Fig. 15} Fig. 15 is a schematic configuration diagram of a fluorescence observation apparatus according to a third embodiment of the present invention.
{Fig. 16} Fig. 16 includes part (a) illustrating a wavelength characteristic of a No-sensitive-probe fluorescence spectrum and part (b) illustrating a wavelength characteristic of a reference-dye fluorescence spectrum.
{Fig. 17} Fig. 17 includes part (a) illustrating a wavelength characteristic of excitation light emitted from a first excitation light source and part (b) illustrating a wavelength characteristic of excitation light emitted from a second excitation light source.
{Fig. 18} Fig. 18 includes part (a) illustrating a reflectance characteristic of a first dichroic mirror and part (b) illustrating a reflectance characteristic of a second dichroic mirror.
{Fig. 19} Fig. 19 includes part (a) illustrating a reflectance characteristic of a first excitation-light cut filter and part (b) illustrating a reflectance characteristic of a second excitation-light cut filter.
{Fig. 20} Fig. 20 includes part (a) illustrating a fluorescence image corresponding to NO-sensitive probes and part (b) illustrating a fluorescence image corresponding to reference probes.
{Fig. 21} Fig. 21 is a schematic configuration diagram of a fluorescence observation apparatus according to a fourth embodiment of the present invention.

### {Description of Embodiments}

### {First Embodiment}

A fluorescence observation apparatus according to a first embodiment of the present invention will be described below with reference to the drawings.

As shown in Fig. 1, a fluorescence observation apparatus 1 according to this embodiment is an endoscopic device and includes an insertion section 10 to be inserted into a body cavity of a biological organism and having an image acquisition unit (image acquisition section) 20 that acquires image information, a position control unit (moving means) 30 that controls the position of the insertion section 10 within the body cavity, a light source unit (illuminating section) 40 that generates illumination light and excitation light to be emitted from a tip 10a of the insertion section 10, a liquid supplying unit 50 that supplies a fluorescent probe solution and a cleaning liquid to be ejected from the tip 10a of the insertion section 10, an overall control unit 60 that controls these units 20, 30, 40, and 50, and a display unit 80 that displays the image acquired by the image acquisition unit 20.

The insertion section 10 has an extremely narrow dimension so that it can be inserted into the body cavity of the biological organism. The insertion section 10 includes the image acquisition unit 20, a light guide 12 that transmits the illumination light and the excitation light received from the light source unit 40 to the tip 10a, and a liquid channel 14 and an air channel 16 that are formed so as to extend in the longitudinal direction. Reference numeral 18 denotes an air supplying device that sends air into the body cavity from the tip 10a via the air channel 16.

For example, as shown in Fig. 2, the image acquisition unit 20 includes an image-acquisition optical system 22 that collects return light or fluorescence received from an inner wall of the body cavity acting as a target photographic object, an excitation-light cut filter 24 that cuts excitation light received from the target photographic object via the image-acquisition optical system 22, a tunable spectroscopic element 26 whose spectral characteristics can be varied by the operation of the overall control unit 60, and an image acquisition element 28 that acquires an image of the return light or the fluorescence collected by the image-acquisition optical system 22 so as to acquire image information.

The tunable spectroscopic element 26 is, for example, an etalon optical filter equipped with two flat optical members 25a and 25b arranged in parallel with a certain distance therebetween and provided with reflective films on opposing surfaces thereof, and an actuator 27 that changes the distance between these optical members 25a and 25b. The tunable spectroscopic element 26 can vary the spectral characteristics, that is, the wavelength range of light to be transmitted therethrough, by changing the distance between the optical members 25a and 25b by the operation of the actuator 27.

The actuator 27 is, for example, a piezoelectric element.

The position control unit 30 is configured to move the insertion section 10 relative to the inner wall of the body cavity of the biological organism. Furthermore, the position control unit 30 includes, for example, a measuring section (not shown) that measures the position of the insertion section 10 within the body cavity by means of a magnetic field, X-rays, or the like.

The light source unit 40 includes a white-light-observation light source 42 that illuminates the inner wall of the body cavity and emits blue, green, and red illumination light rays for acquiring a white-light image, a fluorescence-excitation light source 44 that emits excitation light onto the inner wall within the body cavity so as to excite a fluorescent material existing within the inner wall of the body cavity, thus generating fluorescence, and a light-source control circuit 46 that controls the white-light-observation light source 42 and the fluorescence-excitation light source 44.

The fluorescence-excitation light source 44 is, for example, an argon laser (or a semiconductor laser). An LED (light-emitting diode) or a combination of a xenon lamp and a specific-wavelength-transmission filter may be used as the fluorescence-excitation light source 44.

For example, the illumination light to be emitted from the white-light-observation light source 42 has the wavelength characteristic shown in Fig. 3(a), whereas the excitation light to be emitted from the fluorescence-excitation light source 44 has the wavelength characteristic shown in Fig. 3(b). The excitation-light cut filter 24 has the transmittance characteristic shown in Fig. 3(c). The fluorescence from GST-n-sensitive fluorescent probes generated at the inner wall of the body cavity has, for example, the wavelength characteristic shown in Fig. 3(d).

The light-source control circuit 46 actuates the white-light-observation light source 42 and the fluorescence-excitation light source 44 by switching between them at a predetermined timing.

The liquid supplying unit 50 includes a thermostatic bath 52, a tank for fluorescent probe solution 54A and a tank for cleaning liquid 54B that are disposed within the thermostatic bath 52 and that respectively store the fluorescent probe solution and the cleaning liquid, and a valve 56 that feeds/blocks the liquids from the tank for fluorescent probe solution 54A and the tank for cleaning liquid 54B. Reference numeral 58 denotes a temperature display section that displays the temperature of the thermostatic bath 52.

The thermostatic bath 52 can maintain the liquids in the tank for fluorescent probe solution 54A and the tank for cleaning liquid 54B at a constant temperature.

The valve 56 switches between the tank for fluorescent probe solution 54A and the tank for cleaning liquid 54B so as to connect one of the tanks to the liquid channel 14 of the insertion section 10 in a flowable manner.

For example, a solution in which NO-sensitive fluorescent probes are dissolved, is used as the fluorescent probe solution. The NO-sensitive fluorescent probes hardly generate fluorescence prior to reacting with NO, but have properties by which they change to a material that emits strong fluorescence by reacting with NO existing in an organ within the body cavity.

ACF are known to have an abundance of iNOS occurring therein. When excitation light is emitted onto the inner wall of the body cavity to which the fluorescent probe solution in which the NO-sensitive fluorescent probes are dissolved is sprayed, the NO-sensitive fluorescent probes reacting with NO produced by activation of iNOS in the inner wall of the body cavity are excited, whereby strong fluorescence can be obtained from areas where iNOS exists abundantly, that is, areas where ACF exist.

The overall control unit 60 includes a tunable-spectroscopic-element control circuit 62 that controls the spectral characteristics of the tunable spectroscopic element 26, an image-acquisition-element control circuit 64 that drives and controls the image acquisition element 28, a valve control circuit 66 that controls the opening and the closing of the valve 56 within the liquid supplying unit 50, a frame memory 68 that stores the image information acquired by the image acquisition element 28, and an image-processing and determination device 70 that processes the image information stored in the frame memory 68.

The frame memory 68 includes a white-light-image memory 67A that stores image information (referred to as "white-light image information" hereinafter) acquired by the image acquisition element 28 by taking an image of return light, and a fluorescence-image memory 67B that stores image information (referred to as "fluorescence image information" hereinafter) acquired by the image acquisition element 28 by taking an image of fluorescence. In the fluorescence-image memory 67B, the fluorescence image information may be stored in correspondence with the positional information of the insertion section 10 measured by the position control unit 30.

The tunable-spectroscopic-element control circuit 62 and the image-acquisition-element control circuit 64 are connected with the light-source control circuit 46 of the light source unit 40, and the tunable spectroscopic element 26 and the image acquisition element 28 are driven and controlled in synchronization with the timing at which the light-source control circuit 46 switches between the white-light-observation light source 42 and the fluorescence-excitation light source 44.

Specifically, when R, G, and B illumination light rays are sequentially emitted from the white-light-observation light source 42 by the operation of the light-source control circuit 46, that is, when performing white-light observation based on an RGB frame-sequential method, the spectral characteristics of the tunable spectroscopic element 26 are changed by the operation of the tunable-spectroscopic-element control circuit 62 to spectral characteristics for selectively transmitting the return light. Furthermore, the image-acquisition element 28 is controlled by the operation of the image-acquisition-element control circuit 64 so as to acquire an image of the return light transmitted through the tunable spectroscopic element 26 and to output the acquired white-light image information to the white-light-image memory 67A.

On the other hand, when the excitation light is emitted from the fluorescence-excitation light source 44 by the operation of the light-source control circuit 46, that is, when performing fluorescence observation, the spectral characteristics of the tunable spectroscopic element 26 are changed by the operation of the tunable-spectroscopic-element control circuit 62 to spectral characteristics for selectively transmitting the fluorescence. Furthermore, the image acquisition element 28 is controlled by the operation of the image-acquisition-element control circuit 64 so as to acquire an image of the fluorescence transmitted through the tunable spectroscopic element 26 and to output the acquired fluorescence image information to the fluorescence-image memory 67B.

When performing fluorescence observation, the valve control circuit 66 controls the opening and the closing of the valve 56 so as to spray the fluorescent probe solution stored in the tank for fluorescent probe solution 54A. Furthermore, before spraying the fluorescent probe solution and before performing fluorescence observation after the spraying of the fluorescent probe solution, the valve control circuit 66 controls the opening and the closing of the valve 56 so as to clean the inner wall surface of the body cavity of the biological organism or to spray the cleaning liquid for removing the fluorescent probes remaining on the inner wall surface of the body cavity.

In the case of white-light observation, the image-processing and determination device 70 receives the white-light image information, acquired as a result of the emission of the illumination light, from the white-light-image memory 67A and makes the display unit 80 display the white-light image. On the other hand, in the case of fluorescence observation, the image-processing and determination device 70 receives the fluorescence image information, acquired as a result of the emission of the excitation light, from the fluorescence-image memory 67B and makes the display unit 80 display the fluorescence image.

While the fluorescence image is displayed on the display unit 80, the image-processing and determination device 70 sets a reference line (not shown) that moves together with the image acquisition element 28. The reference line is, for example, an annular line having a predetermined size and centered on the center of the image.

The image-processing and determination device 70 is provided with a counting section 72 that counts the number of fluorescence generation sites included in the fluorescence image information, a fluorescence-intensity measuring section 74 that measures the fluorescence intensity at each fluorescence generation site, and a fluorescence-intensity classifying section 76 that classifies the number of fluorescence generation sites counted by the counting section 72 in accordance with the fluorescence intensity at each fluorescence generation site measured by the fluorescence-intensity measuring section 74.

The counting section 72 is for counting the number of fluorescence generation sites (bright spots) generated from accumulation sites of the NO-sensitive fluorescent probes, specifically, the number of ACF. ACF have, for example, a pattern formed of a group of hollow annular sections with high brightness. The number of ACF counted by the counting section 72, the fluorescence intensity of each ACF measured by the fluorescence-intensity measuring section 74, the classification result of the ACF obtained by the fluorescence-intensity classifying section 76, and the like are displayed on the display unit 80.

The operation of the fluorescence observation apparatus 1 according to this embodiment having the above-described configuration will now be described with reference to a flow chart in Fig. 4.

For example, when observing the inner wall of the body cavity in the rectum of the biological organism acting as a target photographic object by using the fluorescence observation apparatus 1 according to this embodiment, the inside of the rectum is cleaned in a pre-treatment, and the insertion section 10 is subsequently inserted into the rectum until the tip 10a faces the inner wall of the body cavity acting as the target photographic object.

At this time, for example, the air supplying device 18 is actuated so as to send air into the body cavity via the air channel 16, causing the rectum to expand. Furthermore, it is preferable that the position control unit 30 be actuated so as to dispose the insertion section 10 on a central axis in the cross section of the rectum. With this disposition, the observation distance from the image acquisition element 28 to the surrounding inner wall of the body cavity can be maintained in a substantially uniform state.

In this state, the light source unit 40 and the overall control unit 60 are actuated. By the operation of the light-source control circuit 46, the white-light-observation light source 42 is actuated and thus generates illumination light, whereby white-light observation is performed (step S1). The illumination light emitted from the white-light-observation light source 42 is transmitted to the tip 10a of the insertion section 10 via the light guide 12 so as to be emitted onto the target photographic object. The emitted illumination light is reflected at the surface of the target photographic object, and the return light thereof is collected by the image-acquisition optical system 22.

The return light collected by the image-acquisition optical system 22 is transmitted through the excitation-light cut filter 24 before becoming incident on the tunable spectroscopic element 26. In this case, the tunable spectroscopic element 26 is controlled by the operation of the tunable-spectroscopic-element control circuit 62 so as to be set in a mode for selectively transmitting the return light. Consequently, the return light is entirely transmitted through the tunable spectroscopic element 26.

Based on the operation of the image-acquisition-element control circuit 64, the image acquisition element 28 acquires an image of the return light transmitted through the tunable spectroscopic element 26, thereby acquiring white-light image information. The white-light image information acquired by the image acquisition element 28 is stored in the white-light-image memory 67A and is sent to the display unit 80 by the image-processing and determination device 70 so as to be displayed thereon.

Subsequently, when performing fluorescence observation by using the fluorescent probes, the valve 56 is opened by the operation of the valve control circuit 66, whereby the tank for cleaning liquid 54B and the liquid channel 14 are connected to each other. Consequently, the cleaning liquid is sprayed into the rectum of the biological organism, thereby washing off a residue (a deposit such as feces) existing on the inner wall surface of the body cavity (step S2).

After the spraying of the cleaning liquid, the tank for cleaning liquid 54B is switched to the tank for fluorescent probe solution 54A by the operation of the valve control circuit 66, thereby connecting the tank for fluorescent probe solution 54A and the liquid channel 14 to each other and thus spraying the fluorescent probe solution into the rectum (step S3).

After a predetermined time period has elapsed since the spraying of the fluorescent probe solution, the tank for cleaning liquid 54B and the liquid channel 14 are connected to each other again by the operation of the valve control circuit 66, thereby spraying the cleaning liquid into the rectum and thus removing the fluorescent probe solution existing on the inner wall surface of the body cavity (step S4).

After the cleaning process, the operation of the light-source control circuit 46 causes switching from the white-light-observation light source 42 to the fluorescence-excitation light source 44 from which excitation light is emitted, thereby performing fluorescence observation (step S5). The excitation light emitted from the fluorescence-excitation light source 44 is transmitted to the tip 10a of the insertion section 10 via the light guide 12 so as to be emitted onto the target photographic object. Thus, the fluorescent probes penetrated in the inner wall of the body cavity acting as the target photographic object are excited, whereby fluorescence is emitted.

The fluorescence emitted from the target photographic object is collected by the image-acquisition optical system 22 of the image acquisition unit 20 and is transmitted through the excitation-light cut filter 24 before becoming incident on the tunable spectroscopic element 26. In this case, the tunable spectroscopic element 26 is controlled by the operation of the tunable-spectroscopic-element control circuit 62 so as to be switched to a mode for selectively transmitting the fluorescence. Thus, the incident fluorescence is transmitted through the tunable spectroscopic element 26.

Based on the operation of the image-acquisition-element control circuit 64, the image acquisition element 28 acquires an image of the fluorescence transmitted through the tunable spectroscopic element 26, thereby acquiring fluorescence image information. The fluorescence image information acquired by the image acquisition element 28 is stored in the fluorescence-image memory 67B and is sent to the display unit 80 by the image-processing and determination device 70 so as to be displayed thereon.

In the image-processing and determination device 70, the counting section 72 is actuated so as to count the number of fluorescence generation sites included in the fluorescence image information, that is, the number of ACF (step S6). In this case, the insertion section 10 is moved by the position control unit 30 so that the fluorescence image information is acquired over a wide area along the inner wall of the body cavity.

As the insertion section 10 inserted in the rectum is pulled while performing fluorescence observation, that is, as image acquisition is performed while moving the tip 10a away from the deeper side of the rectum with the base end of the insertion section 10 serving as the front side in the moving direction, an image of the target photographic object is displayed on the display unit 80 as if it were moving from the outer side of the image to the inner side, as shown in Fig. 5.

In the counting section 72, when a bright spot (ACF) appearing from the outer side of the image moves toward the inner side of the image and crosses over the annular reference line (in other words, when the bright spot (ACF) enters a predetermined region of the image), the number of bright spots (ACF) that have crossed over the reference line is counted. By performing the counting process in this manner, the number and the positions of the ACF can be readily ascertained while the image acquisition element 28 acquires the image of the inner wall of the body cavity.

With the fluorescence observation apparatus 1 according to this embodiment, when the excitation light is emitted onto the inner wall of the body cavity of the biological organism to which the NO-sensitive fluorescent probe solution is sprayed, the NO-sensitive fluorescent probes reacting with NO existing in the inner wall of the body cavity are excited, whereby strong fluorescence can be obtained from areas where iNOS exists abundantly, that is, areas where ACF exist. Therefore, the counting section 72 can readily and accurately count the number of ACF without miscounting the number of bright spots due to noise or the like or overlooking the existence of ACF.

Subsequently, the fluorescence-intensity measuring section 74 measures the fluorescence intensity of the ACF, the number of which has been counted by the counting section 72, and the fluorescence-intensity classifying section 76 classifies the number of ACF in accordance with the fluorescence intensity of each ACF measured by the fluorescence-intensity measuring section 74. For example, Fig. 6 shows an example of a graph in a case where the inner wall of the body cavity acting as the target photographic object has five ACF with a fluorescence intensity A, seven ACF with a fluorescence intensity B, and five ACF with a fluorescence intensity C.

The degree of malignancy of each ACF can be determined on the basis of the magnitude of the fluorescence intensity of the ACF measured by the fluorescence-intensity measuring section 74. For example, if the fluorescence intensity is high, the degree of malignancy of the ACF is high. By classifying the number of ACF in accordance with the fluorescence intensity in this manner, the future risk for cancer development or the like can be evaluated.

When the entire observation is completed after the ACF classification, the number of the ACF and the like are displayed on the display unit 80 (step S7).

As described above, with the fluorescence observation apparatus 1 according to this embodiment, fluorescence observation is performed over a wide area along the inner wall of the body cavity while moving the insertion section 10, by using the fluorescent probes that react with NO produced by activation of iNOS abundantly occurring in the ACF, so that the number of ACF can be readily and accurately counted without overlooking the existence of ACF, thereby achieving reduced observation time. Furthermore, by classifying the counted number of ACF in accordance with the fluorescence intensity, the future risk for cancer development or the like can be evaluated.

Although the fluorescence intensity at each fluorescence generation site is measured by the fluorescence-intensity measuring section 74 and the number of fluorescence generation sites is classified in accordance with the fluorescence intensity by the fluorescence-intensity classifying section 76 in this embodiment, the image-processing and determination device 70, for example, may alternatively include an image generating section, in place of the fluorescence-intensity measuring section 74, for generating a two-dimensional fluorescence image of bright spots, the number of which is counted by the counting section 72, and a pattern classifying section, in place of the fluorescence-intensity classifying section 76, for classifying the bright spots generated by the image generating section in accordance with the pattern thereof.

In this case, the image generating section may generate the fluorescence image on the basis of the fluorescence image information sent from the fluorescence-image memory 67B and make the display unit 80 display the generated fluorescence image. Furthermore, the counting section 72 may be configured to count not only the number of bright spots but also the number of bright spots classified in accordance with the pattern thereof. Moreover, upon completion of the observation, the pattern of each of the bright spots may be displayed on a screen, an operator may then classify each bright spot in accordance with the pattern thereof, and the classified result may be displayed on the screen in the form of a graph or the like.

It is known that the degree of malignancy of ACF varies depending on its structure. Examples of structures of ACF include type A in which the pit pattern is circular (a near-circular pattern), as shown in Fig. 7(a), type B in which the pit pattern is starfish-like (a near-cross-shaped pattern), as shown in Fig. 7(b), and type C in which the pit pattern is collapsed (a near-linear pattern), as shown in Fig. 7(c). The degree of malignancy increases in the following order: type A < type B < type C.

In this embodiment, although white-light observation is performed by using the white-light-observation light source 42, and the cleaning liquid and the fluorescent probe solution are sprayed by using the liquid supplying unit 50, the white-light-observation light source 42 and the liquid supplying unit 50 may alternatively be omitted, and the cleaning liquid and the fluorescent probe solution may be applied in a pre-treatment prior to performing fluorescence observation by using an alternative member.

In this case, for example, as shown in a flow chart in Fig. 8, after cleaning the rectum using the alternative member (pre-treatment 1), spraying the fluorescent probe solution (pre-treatment 2), and then removing the fluorescent probe solution (pre-treatment 3) as pre-treatment steps, the insertion section 10 may be inserted into the body cavity to perform fluorescence observation (step S5), the number of ACF may be counted (step S6), and the number of ACF may be displayed (step S7).

Furthermore, in this embodiment, for example, a transparent cover that covers the periphery of the insertion section 10 may be provided. In this case, the cover may be fixed to the inner wall of the body cavity inside the rectum so as to maintain the shape of the rectum, and only the insertion section 10 may be moved by the position control unit 30. By using the cover to maintain the shape of the rectum so that no creases or the like are formed in the inner wall of the body cavity, the number of ACF can be accurately counted more readily. It is preferable that the cover be of a disposable type. A balloon-type cover may be used.

Furthermore, for example, the insertion section 10 may be made bendable in a direction orthogonal to the longitudinal direction thereof, so that when a bright spot, such as a fluorescence generation site, is found, the insertion section 10 may be bent so as to set the tip 10a to face the bright spot, thereby performing magnified observation.

The brightness distribution of an image when taken inside a tube (such as a rectum) is, for example, as shown in Fig. 9. In the figure, the ordinate denotes the brightness, whereas the abscissa denotes the distance from the center of the image when the distance from the center to an edge of the image is defined as 1. In Figs. 9 and 10, an image position between 0.8 and 1.0 (i.e., a position far from the center of the image but close to the edge of the image) is susceptible to the structure inside the tube and is in a range where it is difficult to obtain a stable fluorescence signal due to the effect of positional deviation (parallax) between the illuminating position of the tip 10a of the insertion section 10 and the observation optical system. Therefore, counting the number of bright spots when they are positioned between 0.8 and 1.0 results in a large amount of error. On the other hand, a position between 0 and 0.4 (i.e., a position close to the center of the image) provides a weak fluorescence signal and is susceptible to noise since the distance from the tip 10a of the insertion section 10 is far. Therefore, it is preferable to count the number of bright spots when they exist in an area between 0.4 and 0.8.

This embodiment can be modified as follows.

Although each ACF are classified in accordance with the fluorescence intensity by the fluorescence-intensity measuring section 74 and the fluorescence-intensity classifying section 76 in this embodiment, a first modification in which, for example, a fluorescence-size measuring section that measures the size of a fluorescence generation site is used in place of the fluorescence-intensity measuring section 74 and a fluorescence-size classifying section that classifies the number of fluorescence generation sites counted by the counting section 72 in accordance with the size of each fluorescence generation site measured by the fluorescence-size measuring section is used in place of the fluorescence-intensity classifying section 76 is permissible. Since the degree of malignancy of each ACF can be determined in accordance with the size of the ACF, and the number of ACF is classified in accordance with the size thereof, the future risk for cancer development or the like can be evaluated.

According to the invention, a fluorescence-pattern determining section that detects the pattern of a fluorescence generation site and determines whether or not the pattern matches an ACF pattern is used in place of the fluorescence-intensity measuring section 74, and a correcting section that subtracts the number of fluorescence generation sites determined as not satisfying the ACF pattern condition by the fluorescence-pattern determining section from the number of fluorescence generation sites counted by the counting section 72 is used in place of the fluorescence-intensity classifying section 76.

Thus, bright spots, other than ACF, occurring from noise or the like can be removed in accordance with the pattern of each fluorescence generation site, thereby allowing for more accurate detection of ACF. Consequently, by correcting the counted number of fluorescence generation sites so as to accurately ascertain the number of ACF, the future risk for cancer development or the like can be finely evaluated. The ACF pattern condition may be set, for example, on the basis of a ratio between bright areas and dark areas within the pattern of each fluorescence generation site.

Although the image-processing and determination device 70 sets the reference line in the image information and the counting section 72 counts the number of ACF that cross over the reference line in this embodiment, a third modification in which, for example, the image-processing and determination device 70 includes an image combining section that obtains a still composite image by combining multiple sets of image information acquired while moving the insertion section 10, and the counting section 72 counts the number of fluorescence generation sites with predetermined brightness or higher within the still composite image formed by the image combining section is permissible. Thus, based on the still composite image obtained by the image combining section, the number of ACF with a particularly high degree of malignancy can be ascertained in correspondence with the positions and the sizes thereof in an a posteriori manner.

### {Second Embodiment}

Next, a fluorescence observation apparatus according to a second embodiment of the present invention will be described with reference to Fig. 11.

A fluorescence observation apparatus 201 according to this embodiment is a scanning-type endoscopic device that scans an inner wall of a body cavity of a biological organism so as to acquire an image thereof. The fluorescence observation apparatus 201 includes an insertion section 210 that is to be inserted into the body cavity of the biological organism, an illuminating device (illuminating section) 240 that generates excitation light to be emitted from the insertion section 210, a beam splitter 282 that splits the excitation light emitted from the illuminating device 240 and fluorescence generated at the inner wall of the body cavity from each other, a PD (photodetector, image acquisition section) 220 that acquires an image of the fluorescence split by the beam splitter 282 so as to acquire fluorescence image information, and an image-information processing device 270 that processes the fluorescence image information acquired by the PD 220. Reference numeral 284 denotes a first collimator lens.

In the following description, sections with the same configurations as those in the fluorescence observation apparatus 1 according to the first embodiment are given the same reference numerals, and descriptions thereof will be omitted.

The insertion section 210 includes a transparent cover 286 that covers the periphery thereof, a fiber 212 that optically guides the excitation light received from the illuminating device 240 via the beam splitter 282 and the first collimator lens 284 and emits the excitation light from an emission end 212a, a second collimator lens 214, a reflective mirror 216 that reflects the excitation light transmitted through the second collimator lens 214 in a direction orthogonal to the light axis, and a rotating mechanism 218 that rotates the reflective mirror 216.

The image-information processing device 270 includes an image combining section 288 that obtains a still composite image by combining multiple sets of fluorescence image information acquired by the PD 220, a counting section 72, a fluorescence-intensity measuring section 74, and a fluorescence-intensity classifying section 76. The image combining section 288 is configured to obtain the still composite image of the entire inner wall of the body cavity of the rectum on the basis of the position of the insertion section 210 measured by the position control unit 30 and the rotational angle of the reflective mirror 216.

The still composite image obtained by the image combining section 288 is displayed on a display section 280 while being updated during fluorescence observation. The counting section 72 counts the number of ACF existing in the still composite image after the fluorescence observation and displays it on the display section 280. The still composite image may be such that, for example, as shown in Fig. 12, the ordinate thereof denotes the rotational angle of the reflective mirror 216 and the abscissa thereof denotes the position in the rectum in the longitudinal direction thereof from the colon side toward the anus side.

Regarding the fluorescence observation apparatus 201 having such a configuration, the insertion section 210 is inserted into the body cavity to which GST-n-sensitive fluorescent probes are applied in advance, and the excitation light emitted from the illuminating device 240 and transmitted through the beam splitter 282 is optically guided by the fiber 212 so as to be emitted from the emission end 212a. The excitation light emitted from the emission end 212a is reflected by the reflective mirror 216 that is rotated by the operation of the rotating mechanism 218, so as to be emitted in the circumferential direction from a side surface of the insertion section 210 onto the inner wall of the body cavity of the rectum. Then, fluorescence generated at the inner wall of the body cavity is reflected by the reflective mirror 216, is optically guided by the fiber 212, and is split by the beam splitter 282 before becoming incident on the PD 220. Thus, an image of the fluorescence incident on the PD 220 is acquired, whereby fluorescence image information is acquired.

As described above, with the fluorescence observation apparatus 201 according to this embodiment, fluorescence observation is performed by using the reflective mirror 216 and the rotating mechanism 218 to circumferentially scan the inner wall of the body cavity of the rectum and by using the position control unit 30 to move the insertion section 210 in the longitudinal direction thereof relative to the inner wall of the body cavity, whereby image information of the entire inner wall of the body cavity in the rectum can be acquired. Then, based on the still composite image obtained by the image combining section 288, the number of ACF with a particularly high degree of malignancy can be ascertained in correspondence with the positions and the sizes thereof. Furthermore, since there is no need to dispose an image acquisition section within the insertion section 210, the configuration of the insertion section 210 can be simplified.

As an alternative to the fluorescence observation apparatus 201 described in terms of a scanning-type endoscopic device in this embodiment, for example, a fluorescence observation apparatus 401 may be a panorama-type endoscopic device that can acquire an entire circumferential image of the inner wall of the body cavity in the rectum at once, as shown in Fig. 13.

In this case, in place of the second collimator lens 214, the reflective mirror 216, and the rotating mechanism 218, an insertion section 410 may include a conical mirror 416 disposed at a tip 410a of the insertion section 410 and configured to reflect the excitation light, emitted from the illuminating device 240 and optically guided by the fiber 212, in a direction orthogonal to the light axis, and an image acquisition unit (image acquisition section) 420 that acquires an image of fluorescence generated at the inner wall of the body cavity as a result of the emission of the excitation light so as to acquire fluorescence image information. The image acquisition unit 420 may be constituted of an image-acquisition optical system 22, an excitation-light cut filter 24, and a CCD 428.

Regarding the fluorescence observation apparatus 401 having such a configuration, the insertion section 410 is inserted into the body cavity to which NO-sensitive fluorescent probes are applied in advance, and the excitation light emitted from the illuminating device 240 and optically guided by the fiber 212 is reflected by the conical mirror 416 so as to be emitted from a side surface of the insertion section 410 to the entire circumference of the inner wall of the body cavity in the rectum. Fluorescence generated at the inner wall of the body cavity is reflected by the conical mirror 416 so as to become incident on the image acquisition unit 420, whereby sets of fluorescence image information covering the entire circumference of the inner wall of the body cavity in the rectum are sequentially acquired. Then, the image combining section 288, for example, combines multiple panoramic fluorescence images, which display the inner wall in the entire circumference of the body cavity of the rectum, from the colon side toward the anus side of the rectum, as shown in Fig. 14, thereby acquiring a still composite image. In this manner, the fluorescence image information can be acquired within a short time, and the configuration of the insertion section 410 can be further simplified, thereby achieving a narrower shape and weight reduction.

### {Third Embodiment}

Next, a fluorescence observation apparatus according to a third embodiment of the present invention will be described.

A fluorescence observation apparatus 501 according to this embodiment is a scanning-type endoscopic device and is configured to perform fluorescence observation by using a reference dye together with NO-sensitive probes. As shown in Fig. 15, the fluorescence observation apparatus 501 includes an excitation light source (illuminating section) 540A that emits excitation light with a wavelength band of 480 nm, an excitation light source (illuminating section) 540B that emits excitation light (reference excitation light) with a wavelength band of 750 nm, a first dichroic mirror (DM) 581, a second dichroic mirror 583 that splits fluorescence generated by the excitation light from the excitation light source 540A and fluorescence generated by the excitation light from the excitation light source 540B from each other, a first PD (photodetector, image acquisition section) 520A and a second PD (photodetector, image acquisition section) 520B that respectively acquire images of the fluorescence split by the second dichroic mirror (DM) 583 so as to acquire fluorescence image information, and an image-information processing device 570 that processes the fluorescence image information acquired by the first PD 520A and the second PD 520B.

The image-information processing device 570 is provided with a counting section 72, an image generating section 574 that generates two-dimensional fluorescence images on the basis of the fluorescence image information acquired by the first PD 520A and the second PD 520B, and an image comparing section 576 that compares the generated fluorescence images. Reference numeral 519A denotes a first excitation-light cut filter, and reference numeral 519B denotes a second excitation-light cut filter.

In the following description, sections with the same configurations as those in the fluorescence observation apparatus 1 according to the first embodiment or the fluorescence observation apparatus 201 or 401 according to the second embodiment are given the same reference numerals, and descriptions thereof will be omitted.

In this embodiment, for example, the NO-sensitive-probe fluorescence spectrum has the wavelength characteristic shown in Fig. 16(a), and the reference-dye fluorescence spectrum has the wavelength characteristic shown in Fig. 16(b). The excitation light emitted from the excitation light source 540A has the wavelength characteristic shown in Fig. 17(a), and the excitation light emitted from the excitation light source 540B has the wavelength characteristic shown in Fig. 17(b). The first dichroic mirror 581 has the reflectance characteristic shown in Fig. 18(a), and the second dichroic mirror 583 has the reflectance characteristic shown in Fig. 18(b). The first excitation-light cut filter 519A has the reflectance characteristic shown in Fig. 19(a), and the second excitation-light cut filter 519B has the reflectance characteristic shown in Fig. 19(b).

When the NO-sensitive probes are applied to the inner wall of the body cavity of the rectum, there are two kinds of areas where the fluorescence emits with high intensity, i.e., increased iNOS areas (cells with a higher iNOS density than the surrounding areas) and areas where the NO-sensitive probes tends to accumulate easily. For example, when there are dead cells (i.e., cells detached from the rectum surface) or the like, the NO-sensitive probes may sometimes get trapped in these dead cells and emit. Since iNOS also exists in normal areas and produces NO, it is not only the ACF that emit. ACF emit fluorescence easily simply because there is a large amount of iNOS therein, and when there is a large amount of NO-sensitive probes accumulated in other areas, fluorescence emitting with high intensity is also observed in these dye-accumulated areas. For example, an inflamed area or the like may sometimes emit since the dye itself can easily penetrate the cells thereof.

Regarding the fluorescence observation apparatus 501 having such a configuration, the NO-sensitive probes are applied to the inside of the body cavity together with the reference dye (IR-780) and are then removed after several minutes, and subsequently, excitation light rays are simultaneously emitted from the excitation light source 540A and the excitation light source 540B onto the inner wall of the body cavity. Fluorescence generated at the inner wall of the body cavity is optically guided by the fiber 212 and is reflected by the beam splitter 282 before being split by the second dichroic mirror 583. The fluorescence from the NO-sensitive probes travels through the first excitation-light cut filter 519A before an image of the fluorescence is acquired by the first PD 520A, whereas the fluorescence from reference probes travels through the second excitation-light cut filter 519B before an image of the fluorescence is acquired by the second PD 520.

In the image-information processing device 570, the image generating section 574 generates a fluorescence image, as shown in Fig. 20(a), on the basis of the fluorescence image information acquired by the first PD 520A and also generates a fluorescence image, as shown in Fig. 20(b), on the basis of the fluorescence image information acquired by the second PD 520B.

As shown in Fig. 20(a), the fluorescence image (referred to as "fluorescence image A" hereinafter) corresponding to the NO-sensitive probes has two kinds of bright spots, that is, bright spots of ACF and bright spots of noise (i.e., areas where the dye tends to accumulate easily). On the other hand, as shown in Fig. 20(b), the fluorescence image (referred to as "fluorescence image B" hereinafter) corresponding to the reference probes only has bright spots of noise. The image comparing section 576 compares the fluorescence image A and the fluorescence image B with each other, and the counting section 72 counts the number of bright spots existing only in the fluorescence image A so that only the number of ACF can be counted.

Although the image-information processing device 570 includes the image comparing section 576 in this embodiment, for example, the image comparing section 576 may be omitted, and the image generating section 574 may be configured to perform correction, such as subtraction processing or division processing, on the fluorescence image A on the basis of the fluorescence image B. In this manner, the fluorescence image can be made to have noise removed therefrom so as to have only ACF therein, whereby the number of ACF can be accurately counted.

### {Fourth Embodiment}

Next, a fluorescence observation apparatus according to a fourth embodiment of the present invention will be described.

As shown in Fig. 21, a fluorescence observation apparatus 301 according to this embodiment is a capsule-type endoscopic device and has a capsule sheath 390 formed in a capsule shape that encapsulates an illuminating device 240, a conical mirror 416, an image acquisition unit 420, an image-information processing device 270, a wireless unit 392 that sends fluorescence image information acquired by the image acquisition unit 420 to the outside, a permanent magnet 393, and a battery 394 that supplies power to the illuminating device 240 and the like.

In the following description, sections with the same configurations as those in the fluorescence observation apparatus 1 according to the first embodiment, the fluorescence observation apparatus 201 or 401 according to the second embodiment, or the fluorescence observation apparatus 501 according to the third embodiment are given the same reference numerals, and descriptions thereof will be omitted.

The permanent magnet 393 has, for example, a columnar shape with its column halves magnetized to an N-pole and an S-pole, respectively, and the magnetic poles are fixed in a direction orthogonal to the longitudinal axis of the capsule shape.

The outer peripheral surface of the capsule sheath 390 is provided with an optical window 396 formed along the periphery in the longitudinal direction and a propelling mechanism (moving means) 397 formed of a wire, circular in cross section, helically wound around the longitudinal axis. Emission of excitation light and detection of fluorescence are performed via the optical window 396. The propelling mechanism 397 converts rotation of the fluorescence observation apparatus 301 around the longitudinal axis into propelling motion.

The operation of the fluorescence observation apparatus 301 having such a configuration will now be described.

The fluorescence observation apparatus 301 is inserted through the anus and into the rectum to which NO-sensitive fluorescent probes are applied in advance, and is sent to the sigmoid. When an external magnetic field rotating around the longitudinal axis is applied to the fluorescence observation apparatus 301, the propelling mechanism 397 causes the fluorescence observation apparatus 301 to move linearly while rotating around the longitudinal axis.

As the fluorescence observation apparatus 301 moves inside the rectum, excitation light emitted from the illuminating device 240 is transmitted through the optical window 396 so as to be emitted onto the inner wall of the body cavity, and fluorescence generated at the inner wall of the body cavity is reflected by a conical mirror 398 via the optical window 396 so as to become incident on the image acquisition unit 420. Fluorescence image information of the inner wall of the body cavity is acquired by the image acquisition unit 420 and is image-processed in the image-information processing device 270.

A counting section 72 counts the number of ACF, and the wireless unit 392 continuously wirelessly-transmits the fluorescence image information, the counted-number result, and the like to a receiver (not shown) disposed outside the biological organism. The fluorescence observation apparatus 301 is expelled outward from the biological organism through the anus. For example, an external device (not shown) disposed outside the biological organism may display the ACF by retrieving the fluorescence image information from the receiver.

The fluorescence observation apparatus 301 may include a storage section that stores the fluorescence image information acquired by the image acquisition unit 420 so that, after the fluorescence observation apparatus 301 is expelled from the body cavity, the fluorescence image information can be transmitted to the receiver from the wireless unit 392 or the fluorescence image information can be transferred to the external device by removing the storage section from the capsule sheath 390.

Although the counting section 72 is disposed within the capsule sheath 390 in this embodiment, a capsule endoscopic system may be formed by disposing the counting section 72 in the external device.

Although the embodiments of the present invention have been described above in detail with reference to the drawings, specific configurations are not to be limited to the embodiments, and the present invention may include design modifications so long as they do not depart from the scope of the invention. For

Furthermore, for example, although NO-sensitive fluorescence probes are explained as an example of fluorescence probes which make areas where ACF exists abundantly emits strong light, iNOS-sensitive fluorescence probes, whose characteristics change from weakly fluorescent to strongly fluorescent by activation of iNOS, may be employed.

Furthermore, for example, although each ACF are classified in accordance with the fluorescence intensity, the size, or the pattern in each of the above embodiments, the ACF may be classified in accordance with a combination of the fluorescence intensity, the size, and the pattern.

### {Reference Signs List}

- 1, 201, 301, 401, 501: fluorescence observation apparatus
- 20,: 420 image acquisition unit (image acquisition section)
- 30: position control unit (moving means)
- 40: light source unit (illuminating section)
- 72: counting section
- 220: PD (image acquisition section)
- 240: illuminating device (illuminating section)
- 397: propelling mechanism (moving means)
- 574: image generating section

## Claims

1. A fluorescence observation apparatus (1, 201, 301, 401, 501) comprising:
an illuminating section (40) that is adapted to be inserted into a body cavity of a biological organism and to emit excitation light onto an inner wall of the body cavity;
an image acquisition section (20, 420) that is adapted to acquire an image of fluorescence generated by the excitation light emitted from the illuminating section (40) so as to acquire image information; and
a fluorescent-probe discharging section that stores fluorescent probes adapted so that fluorescence characteristics change by reacting with iNOS existing in the inner wall of the body cavity or so that fluorescence characteristics change by reacting with NO produced by activation of iNOS, wherein the fluorescent probes are adapted to be excited so as to generate the fluorescence generation sites, the fluorescent-probe discharging section being adapted to spray the fluorescent probes to the inner wall of the body cavity,
**characterized in that** the fluorescence observation apparatus (1, 201, 301, 401, 501) further comprises:
a counting section (72) that is adapted to count the number of fluorescence generation sites on the basis of the fluorescence included in the image information acquired by the image acquisition section (20, 420);
a fluorescence-pattern determining section that is adapted to detect a pattern of each fluorescence generation site and determine whether or not the pattern matches a pattern of aberrant crypt foci, ACF; and
a correcting section that is adapted to subtract the number of fluorescence generation sites determined as not satisfying a pattern condition of the ACF by the fluorescence-pattern determining section from the number of fluorescence generation sites counted by the counting section (72).

2. The fluorescence observation apparatus (1, 201, 301, 401, 501) according to Claim 1, further comprising moving means (397) adapted to move the illuminating section (40) and the image acquisition section (20,420) relative to the inner wall of the body cavity.

3. The fluorescence observation apparatus (1, 201, 301, 401, 501) according to Claim 1, wherein the counting section (72) is adapted to count the number of fluorescence generation sites existing in an area between 0.4 and 0.8 from the center of the image based on the image information acquired by the image acquisition section (20, 420) when the distance from the center to an edge of the image is defined as 1.

4. The fluorescence observation apparatus (1, 201, 301, 401, 501) according to Claim 1, further comprising:
a fluorescence-intensity measuring section (74) that is adapted to measure the fluorescence intensity at each fluorescence generation site; and
a fluorescence-intensity classifying section (76) that is adapted to classify the number of fluorescence generation sites counted by the counting section (72) in accordance with the fluorescence intensity at each fluorescence generation site measured by the fluorescence-intensity measuring section (74).

5. The fluorescence observation apparatus (1, 201, 301, 401, 501) according to Claim 1, further comprising:
a fluorescence-size measuring section that is adapted to measure the size of each fluorescence generation site; and
a fluorescence-size classifying section that is adapted to classify the number of fluorescence generation sites counted by the counting section (72) in accordance with the size of each fluorescence generation site measured by the fluorescence-size measuring section.

6. The fluorescence observation apparatus (1, 201, 301, 401, 501) according to Claim 1, further comprising:
an image generating section (574) that is adapted to generate a two-dimensional image of the fluorescence generation sites counted by the counting section (72); and
a pattern classifying section that is adapted to classify each fluorescence generation site in accordance with a pattern thereof on the basis of the two-dimensional image generated by the image generating section (574).

7. The fluorescence observation apparatus (1, 201, 301, 401, 501) according to Claim 1, wherein the illuminating section (40) is adapted to emit reference excitation light having a wavelength characteristic different from that of the excitation light, and
wherein the fluorescence observation apparatus (1, 201, 301, 401, 501) further comprises an image generating section (574) that is adapted to generate a two-dimensional fluorescence image and a two-dimensional reference fluorescence image on the basis of the image information corresponding to the excitation light and image information corresponding to the reference excitation light that are acquired by the image acquisition section (20, 420), and that is adapted to correct the fluorescence image acquired using the excitation light by using the reference fluorescence image acquired using the reference excitation light.

8. The fluorescence observation apparatus (1, 201, 301,401, 501) according to any one of Claims 2 to 7, further comprising an image combining section (288) that is adapted to obtain a still composite image by combining multiple sets of image information acquired by the image acquisition section (20, 420) while moving the image acquisition section (20, 420) using the moving means (397),
wherein the counting section (72) is adapted to count the number of fluorescence generation sites with predetermined brightness or higher within the still composite image obtained by the image combining section (288).

9. The fluorescence observation apparatus (1, 201, 301, 401, 501) according to any one of Claims 2 to 7, further comprising a reference line that is provided within the image information acquired by the image acquisition section (20, 420) and that moves together with the image acquisition section (20, 420),
wherein when the image acquisition section (20, 420) is moved by the moving means (397), the counting section (72) is adapted to count the number of fluorescence generation sites that cross over the reference line within the image information acquired by the image acquisition section (20, 420).

## Patentansprüche

1. Fluoreszenzbeobachtungsgerät (1, 201, 301, 401, 501), das umfasst:
einen Beleuchtungsabschnitt (40), der dazu eingerichtet ist, in einen Körperhohlraum eines biologischen Organismus eingeführt zu werden und Anregungslicht auf eine Innenwand des Körperhohlraums zu emittieren;
einen Bildaufnahmeabschnitt (20, 420), der dazu eingerichtet ist, ein Bild von Fluoreszenz aufzunehmen, die durch das von dem Beleuchtungsabschnitt (40) emittierte Anregungslicht erzeugt wird, um eine Bildinformation aufzunehmen; und
einen Fluoreszenzsondenabgabeabschnitt, der Fluoreszenzsonden speichert, die so beschaffen sind, dass sich Fluoreszenzcharakteristiken durch eine Reaktion mit iNOS, das in der Innenwand des Körperhohlraums existiert, ändern oder dass sich Fluoreszenzcharakteristiken durch eine Reaktion mit NO, das durch eine Aktivierung von iNOS gebildet wird, ändern, wobei die Fluoreszenzsonden dazu eingerichtet sind, angeregt zu werden, um Fluoreszenzerzeugungsorte zu erzeugen, wobei der Fluoreszenzsondenabgabeabschnitt dazu eingerichtet ist, die Fluoreszenzsonden zu der Innenwand des Körperhohlraums zu sprühen,
**dadurch gekennzeichnet, dass** das Fluoreszenzbeobachtungsgerät (1, 201, 301, 401, 501) ferner umfasst:
einen Zählabschnitt (72), der dazu eingerichtet ist, die Anzahl von Fluoreszenzerzeugungsorten auf der Basis der Fluoreszenz zu zählen, die in der durch den Bildaufnahmeabschnitt (20, 420) aufgenommenen Bildinformation enthalten ist;
einen Fluoreszenzmusterbestimmungsabschnitt, der dazu eingerichtet ist, ein Muster eines jeden Fluoreszenzerzeugungsorts zu erfassen und zu bestimmen, ob das Muster zu einem Muster von anormalen Kryptherden (aberrant crypt foci), ACF, passt oder nicht; und
einen Korrigierabschnitt, der dazu eingerichtet ist, die Anzahl von Fluoreszenzerzeugungsorten, für die durch den Fluoreszenzmusterbestimmungsabschnitt bestimmt wurde, dass sie eine Musterbedingung der ACF nicht erfüllen, von der Anzahl der durch den Zählabschnitt (72) gezählten Fluoreszenzerzeugungsorten zu subtrahieren.

2. Fluoreszenzbeobachtungsgerät (1, 201, 301, 401, 501) gemäß Anspruch 1, das ferner ein Bewegungsmittel (397) umfasst, das dazu eingerichtet ist, den Beleuchtungsabschnitt (40) und den Bildaufnahmeabschnitt (20, 420) relativ zu der Innenwand des Körperhohlraums zu bewegen.

3. Fluoreszenzbeobachtungsgerät (1, 201, 301, 401, 501) gemäß Anspruch 1, wobei der Zählabschnitt (72) dazu eingerichtet ist, auf der Basis der durch den Bildaufnahmeabschnitt (20, 420) aufgenommenen Bildinformation die Anzahl von Fluoreszenzerzeugungsorten zu zählen, die in einem Bereich zwischen 0,4 und 0,8 von einem Mittelpunkt des Bilds existieren, wenn der Abstand von dem Mittelpunkt zu einem Rand des Bilds als 1 definiert ist.

4. Fluoreszenzbeobachtungsgerät (1, 201, 301, 401, 501) gemäß Anspruch 1, das ferner umfasst:
einen Fluoreszenzintensitätsmessabschnitt (74), der dazu eingerichtet ist, die Fluoreszenzintensität an jedem Fluoreszenzerzeugungsort zu messen; und
einen Fluoreszenzintensitätsklassifizierungsabschnitt (76), der dazu eingerichtet ist, die Anzahl von durch den Zählabschnitt (72) gezählten Fluoreszenzerzeugungsorten gemäß der durch den Fluoreszenzintensitätsmessabschnitt (74) gemessenen Fluoreszenzintensität an jedem Fluoreszenzerzeugungsort zu klassifizieren.

5. Fluoreszenzbeobachtungsgerät (1, 201, 301, 401, 501) gemäß Anspruch 1, das ferner umfasst:
einen Fluoreszenzgrößenmessabschnitt, der dazu eingerichtet ist, die Größe eines jeden Fluoreszenzerzeugungsorts zu messen; und
einen Fluoreszenzgrößenklassifizierungsabschnitt, der dazu eingerichtet ist, die Anzahl von durch den Zählabschnitt (72) gezählten Fluoreszenzerzeugungsorten gemäß der durch den Fluoreszenzgrößenmessabschnitt (74) gemessenen Fluoreszenzgröße eines jeden Fluoreszenzerzeugungsorts zu klassifizieren.

6. Fluoreszenzbeobachtungsgerät (1, 201, 301, 401, 501) gemäß Anspruch 1, das ferner umfasst:
einen Bilderzeugungsabschnitt (574), der dazu eingerichtet ist, ein zweidimensionales Bild der durch den Zählabschnitt (72) gezählten Fluoreszenzerzeugungsorte zu erzeugen; und
einen Musterklassifizierungsabschnitt, der dazu eingerichtet ist, jeden Fluoreszenzerzeugungsort gemäß dessen Muster auf der Basis des von dem Bilderzeugungsabschnitt (574) erzeugten zweidimensionalen Bilds zu klassifizieren.

7. Fluoreszenzbeobachtungsgerät (1, 201, 301, 401, 501) gemäß Anspruch 1, wobei der Beleuchtungsabschnitt (40) dazu eingerichtet ist, Referenzanregungslicht mit einer Wellenlängencharakteristik zu emittieren, die sich von der des Anregungslichts unterscheidet, und
wobei das Fluoreszenzbeobachtungsgerät (1, 201, 301, 401, 501) ferner einen Bilderzeugungsabschnitt (574) umfasst, der dazu eingerichtet ist, auf der Basis der dem Anregungslicht entsprechenden Bildinformation und der dem Referenzanregungslicht entsprechenden Bildinformation, die von dem Bildaufnahmeabschnitt (20, 420) aufgenommen werden, ein zweidimensionales Fluoreszenzbild und ein zweidimensionales Referenzfluoreszenzbild zu erzeugen, und der dazu eingerichtet ist, das unter Verwendung des Anregungslichts aufgenommene Fluoreszenzbild durch Verwendung des unter Verwendung des Referenzanregungslichts aufgenommenen Fluoreszenzbilds zu korrigieren.

8. Fluoreszenzbeobachtungsgerät (1, 201, 301, 401, 501) gemäß einem der Ansprüche 2 bis 7, das ferner einen Bildkombinationsabschnitt (288) umfasst, der dazu eingerichtet ist, durch Kombinieren mehrerer Sätze von Bildinformationen, die durch den Bildaufnahmeabschnitt (20, 420) aufgenommen werden, während der Bildaufnahmeabschnitt (20, 420) unter Verwendung des Bewegungsmittels (397) bewegt wird, ein zusammengesetztes Standbild zu erhalten,
wobei der Zählabschnitt (72) dazu eingerichtet ist, die Anzahl von Fluoreszenzerzeugungsorten mit einer vorbestimmten Helligkeit oder höher in dem durch den Bildkombinationsabschnitt (288) erhaltenen zusammengesetzten Standbild zu zählen.

9. Fluoreszenzbeobachtungsgerät (1, 201, 301, 401, 501) gemäß einem der Ansprüche 2 bis 7, das ferner eine Referenzlinie umfasst, die in der durch den Bildaufnahmeabschnitt (20, 420) aufgenommenen Bildinformation vorgesehen ist und die sich gemeinsam mit dem Bildaufnahmeabschnitt (20, 420) bewegt,
wobei, wenn der Bildaufnahmeabschnitt (20, 420) durch das Bewegungsmittel (397) bewegt wird, der Zählabschnitt (72) dazu eingerichtet ist, die Anzahl von Fluoreszenzerzeugungsorten zu zählen, die die Referenzlinie in der durch den Bildaufnahmeabschnitt (20, 420) aufgenommenen Bildinformation überqueren.

## Revendications

1. Appareil (1, 201, 301, 401, 501) d'observation de fluorescence comprenant :
une section (40) d'éclairage qui est adaptée à être insérée dans une cavité corporelle d'un organisme biologique et à émettre une lumière d'excitation sur une paroi intérieure de la cavité corporelle ;
une section (20, 420) d'acquisition d'image qui est adaptée à acquérir une image d'une fluorescence générée par la lumière d'excitation émise par la section (40) d'éclairage de façon à acquérir une information d'image ; et
une section de décharge de sondes fluorescentes qui stocke des sondes fluorescentes adaptées de telle manière que des caractéristiques de fluorescence changent par réaction avec l'iNOS existant dans la paroi intérieure de la cavité corporelle ou de telle manière que des caractéristiques de fluorescence changent par réaction avec le NO produit par activation de l'iNOS, dans lequel les sondes fluorescentes sont adaptées à être excitées de façon à générer les sites de génération de fluorescence, la section de décharge de sondes fluorescentes étant adaptée à pulvériser les sondes fluorescentes sur la paroi intérieure de la cavité corporelle,
**caractérisé en ce que** l'appareil (1, 201, 301, 401, 501) d'observation de fluorescence comprend en outre :
une section (72) de comptage qui est adaptée à compter le nombre de sites de génération de fluorescence sur la base de la fluorescence incluse dans l'information d'image acquise par la section (20, 420) d'acquisition d'image ;
une section de détermination de motif de fluorescence qui est adaptée à détecter un motif de chaque site de génération de fluorescence et déterminer si le motif correspond ou non à un motif de foyers de cryptes aberrantes, FCA ; et
une section de correction qui est adaptée à soustraire le nombre de sites de génération de fluorescence déterminés comme ne satisfaisant pas à une condition de motif des FCA par la section de détermination de motif de fluorescence du nombre de sites de génération de fluorescence comptés par la section (72) de comptage.

2. Appareil (1, 201, 301, 401, 501) d'observation de fluorescence selon la revendication 1, comprenant en outre un moyen (397) de déplacement adapté à déplacer la section (40) d'éclairage et la section (20, 420) d'acquisition d'image par rapport à la paroi intérieure de la cavité corporelle.

3. Appareil (1, 201, 301, 401, 501) d'observation de fluorescence selon la revendication 1, dans lequel la section (72) de comptage est adaptée à compter le nombre de sites de génération de fluorescence existant dans une zone entre 0,4 et 0,8 du centre de l'image sur la base de l'information d'image acquise par la section (20, 420) d'acquisition d'image lorsque la distance du centre à un bord de l'image est définie comme 1.

4. Appareil (1, 201, 301, 401, 501) d'observation de fluorescence selon la revendication 1, comprenant en outre :
une section (74) de mesure d'intensité de fluorescence qui est adaptée à mesurer l'intensité de fluorescence au niveau de chaque site de génération de fluorescence ; et
une section (76) de classement d'intensité de fluorescence qui est adaptée à classer le nombre de sites de génération de fluorescence comptés par la section (72) de comptage en fonction de l'intensité de fluorescence au niveau de chaque site de génération de fluorescence mesurée par la section (74) de mesure d'intensité de fluorescence.

5. Appareil (1, 201, 301, 401, 501) d'observation de fluorescence selon la revendication 1, comprenant en outre :
une section de mesure de taille de fluorescence qui est adaptée à mesurer la taille de chaque site de génération de fluorescence ; et
une section de classement de taille de fluorescence qui est adaptée à classer le nombre de sites de génération de fluorescence comptés par la section (72) de comptage en fonction de la taille de chaque site de génération de fluorescence mesurée par la section de mesure de taille de fluorescence.

6. Appareil (1, 201, 301, 401, 501) d'observation de fluorescence selon la revendication 1, comprenant en outre :
une section (574) de génération d'image qui est adaptée à générer une image bidimensionnelle des sites de génération de fluorescence comptés par la section (72) de comptage ; et
une section de classement de motif qui est adaptée à classer chaque site de génération de fluorescence en fonction d'un motif de celui-ci sur la base de l'image bidimensionnelle générée par la section (574) de génération d'image.

7. Appareil (1, 201, 301, 401, 501) d'observation de fluorescence selon la revendication 1, dans lequel la section (40) d'éclairage est adaptée à émettre une lumière d'excitation de référence ayant une longueur d'onde caractéristique différente de celle de la lumière d'excitation, et
dans lequel l'appareil (1, 201, 301, 401, 501) d'observation de fluorescence comprend en outre une section (574) de génération d'image qui est adaptée à générer une image bidimensionnelle de fluorescence et une image bidimensionnelle de fluorescence de référence sur la base de l'information d'image correspondant à la lumière d'excitation et de l'information d'image correspondant à la lumière d'excitation de référence qui sont acquises par la section (20, 420) d'acquisition d'image, et qui est adaptée à corriger l'image de fluorescence acquise en utilisant la lumière d'excitation en utilisant l'image de fluorescence de référence acquise en utilisant la lumière d'excitation de référence.

8. Appareil (1, 201, 301, 401, 501) d'observation de fluorescence selon l'une quelconque des revendications 2 à 7, comprenant en outre une section (288) de combinaison d'images qui est adaptée à obtenir une image composite fixe en combinant des ensembles multiples d'informations d'images acquis par la section (20, 420) d'acquisition d'image tout en déplaçant la section (20, 420) d'acquisition d'image en utilisant le moyen (397) de déplacement,
dans lequel la section (72) de comptage est adaptée à compter le nombre de sites de génération de fluorescence avec une luminosité prédéterminée ou supérieure au sein de l'image composite fixe obtenue par la section (288) de combinaison d'images.

9. Appareil (1, 201, 301, 401, 501) d'observation de fluorescence selon l'une quelconque des revendications 2 à 7, comprenant en outre une ligne de référence qui est prévue au sein de l'information d'image acquise par la section (20, 420) d'acquisition d'image et qui se déplace en même temps que la section (20,420) d'acquisition d'image,
dans lequel, lorsque la section (20, 420) d'acquisition d'image est déplacée par le moyen (397) de déplacement, la section (72) de comptage est adaptée à compter le nombre de sites de génération de fluorescence qui traversent la ligne de référence au sein de l'information d'image acquise par la section (20, 420) d'acquisition d'image.
